# EUROPEAN PATENT SPECIFICATION

(11) **EP 0 292 398 B1**
(45) Date of publication and mention of the grant of the patent: **19.01.1994**
(21) Application number: 88401224.6
(22) Date of filing: 19.05.1988
(51) Int. Cl.: A61M 1/18, A61M 1/36

(54) **Extracorporeal blood circulating apparatus**
Gerät für die extrakorporale Blutzirkulation
Appareil pour la circulation extracorporelle du sang

(30) Priority: 19.05.1987 JP 122243/87; 29.12.1987 JP 333257/87
(43) Date of publication of application: 23.11.1988
(73) Proprietor: TERUMO KABUSHIKI KAISHA, Tokyo 151 (JP)
(72) Inventor: Katsura, Yoshiro, Fuji-shi Shizuoka-ken (JP)
(74) Representative: Joly, Jean-Jacques

(56) References cited:
- FR-A- 2 525 476

## Description

### BACKGROUND OF THE INVENTION

The present invention relates to an extracorporeal blood circulating apparatus.

When a thoracic operation, for example, is to be carried out on a patient, an extracorporeal blood circulating circuit is established using an artificial lung in which the blood is circulated for an exchange of carbon dioxide and oxygen. The blood is controlled at a prescribed temperature by a heat exchanger, and thereafter the gases are exchanged in the artificial lung. Then, the blood is temporarily stored in a blood reservoir for a steady supply of the blood. The blood is pumped into the patient under the operation at a constant pulse rate.

Blood reservoirs for use in extracorporeal blood circulation include a closed-type blood reservoir in the form of a soft bag for storing blood in an airtight condition and an open-type blood reservoir in the form of a hard housing for storing blood. The open-type blood reservoir is advantageous in that priming and confirmation of the stored amount of blood can easily be performed, and it would be easy to construct the blood reservoir as a unitary component of an artificial lung. Therefore, various extracorporeal blood circulating apparatus employing open-type blood reservoirs have been proposed.

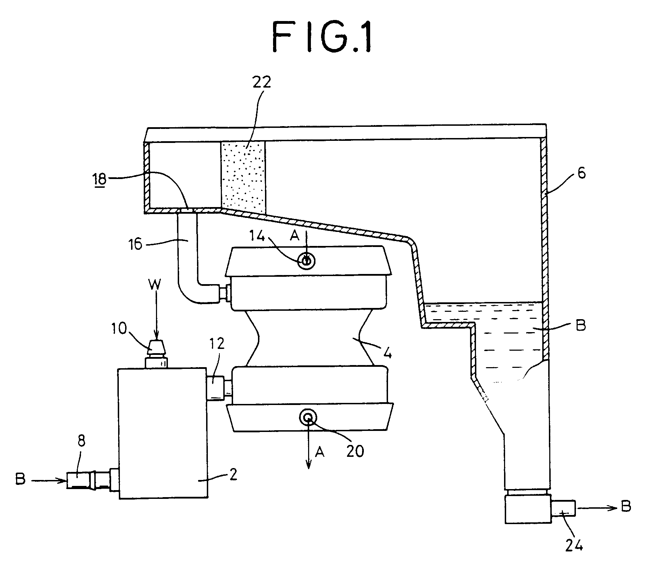

FIG. 1 of the accompanying drawings schematically illustrates a prior art extracorporeal blood circulating apparatus by way of comparative example. The extracorporeal blood circulating apparatus comprises a heat exchanger 2, an artificial lung 4, and a blood reservoir 6 as an interconnected unitary system. The extracorporeal blood circulating apparatus is connected to the body of a patient through a pump (not shown) which supplies blood at a certain pulse rate. Blood B discharged from the human body is introduced from a blood inlet port 8 into the heat exchanger 2 and controlled at a prescribed temperature by warm or cold water supplied from a water inlet port 10. Thereafter, the blood B is fed through a joint tube 12 into the artificial lung 4. The artificial lung 4 contains a multiplicity of hollow filamentary membranes through which a gas A containing oxygen supplied from a gas inlet port 14 flows. The blood B, while passing around the hollow filamentary membranes, receives oxygen from the gas A and discharges carbon dioxide, and then flows via a joint tube 16 through a blood inlet port 18 into the blood reservoir 6. After the exchange of oxygen and carbon dioxide, the gas A is discharged from the artificial lung 4 through a gas outlet port 20. The blood B that has entered the blood reservoir 6 from the blood inlet port 18 goes through an anti-foaming member 22 and is temporarily stored in the blood reservoir 6. Then, the stored blood B is supplied from a blood outlet port 24 into the human body by the pump.

With the above extracorporeal blood circulating apparatus, the blood B is circulated between the apparatus and the human body by the pressure developed by the pump. Where the heat exchanger 2 is vertically elongate as shown in FIG. 1, the blood B flows into the heat exchanger 2 through the blood inlet port 8 at the lower end of the heat exchanger 2 and goes upwardly through the heat exchanger 2, during which time the temperature of the blood B is regulated. Thereafter, the blood B is fed from the joint tube 12 at the upper end of the heat exchanger 2 to the artificial lung 4. The joint tube 16 interconnecting the artificial lung 4 and the blood reservoir 6 is higher in position above ground by the difference in height between the blood inlet port 8 and the joint tube 12. Therefore, the difference in height between the patient and the joint tube 16 is reduced by the difference in height between the blood inlet port 8 and the joint tube 12, resulting in a loss of potential energy. If the blood B were forced to flow from the heat exchanger 2 to the artifical lung 4 only under the blood head arising from the difference in height between the patient and the joint tube 16 without using any pump, no sufficient pressure might be produced for such blood feed. To avoid this problem, the operating table on which the patient lies must be elevated to obtain a desired blood head. If it is impossible to elevate the operating table, then a pump must be connected to the blood inlet port 8 of the heat exchanger 2.

An extracorporeal blood circulating apparatus corresponding to the preamble of claim 1 is also known from FR-A-2 535 476.

### SUMMARY OF THE INVENTION

It is a primary object of the present invention to provide an extracorporeal blood circulating apparatus for controlling the temperature of blood in a heat exchanger, thereafter exchanging gases in the blood with an artificial lung, and then storing the blood in a blood reservoir, the apparatus being capable of delivering the blood in the blood reservoir under the blood head only and also being simple in structure.

According to the invention, the apparatus comprises a heat exchanger having a blood inlet and a blood outlet the blood outlet of the heat exchanger being held in communication with the blood inlet of the artificial lung, the arrangement being such that blood is introduced into the heat exchanger from the blood inlet thereof for temperature control, then delivered from the blood outlet of the heat exchanger into the artificial lung via the blood inlet thereof for a gas exchange in the artificial lung, and thereafter fed out of the blood outlet of the artificial lung.

The characteristic features of the invention are that: the blood inlet of the heat exhanger is in a lower position thereof and said blood outlet is in an upper portion thereof; the blood inlet of the artificial lung is in a lower portion thereof and the blood inlet in an upper portion thereof; and the height of the heat exchanger is smaller than the other dimensions so as to make the height difference between the incoming blood and the outgoing blood small.

The heat exchanger preferably comprises a substantially tubular housing having first and second ends and a plurality of heat exchanger pipes disposed in the housing, with the heat exchanger pipes extending longitudinally in the housing.

In a particular embodiment of the invention, the blood inlet of the heat exchanger is disposed substantially centrally in the lower portion thereof in the longitudinal direction of the heat exchanger, and the blood outlet is disposed in the upper portion on each of opposite ends in the longitudinal direction of the heat exchanger.

The heat exchanger pipes are preferably arranged to be supplied with a temperature control fluid from the first end of the heat exchanger and discharge the temperature control fluid from the second end of the heat exchanger.

The above and other features and advantages of the present invention will become more apparent from the following description when taken in conjunction with the accompanying drawings in which a preferred embodiment of the present invention is shown by way of an illustrative example.

### BRIEF DESCRIPTION OF THE DRAWINGS

FIG. 1 is a schematic elevational view, partly in cross section, of an extracorporeal blood circulating apparatus shown as a comparative example;
FIG. 2 is a schematic elevational view, partly in cross section, of an extracorporeal blood circulating apparatus according to the present invention; and
FIG. 3 is a schematic perspective view of the extracorporeal blood circulating apparatus illustrated in FIG. 2.

### DETAILED DESCRIPTION OF THE PREFERRED EMBODIMENT

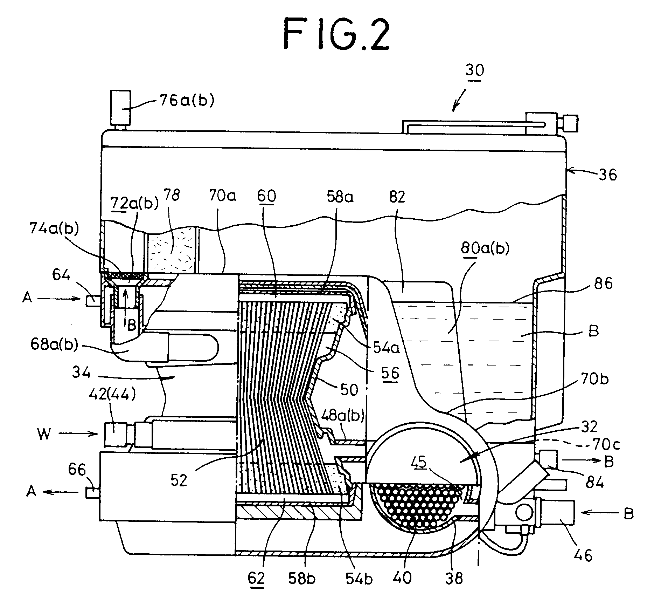

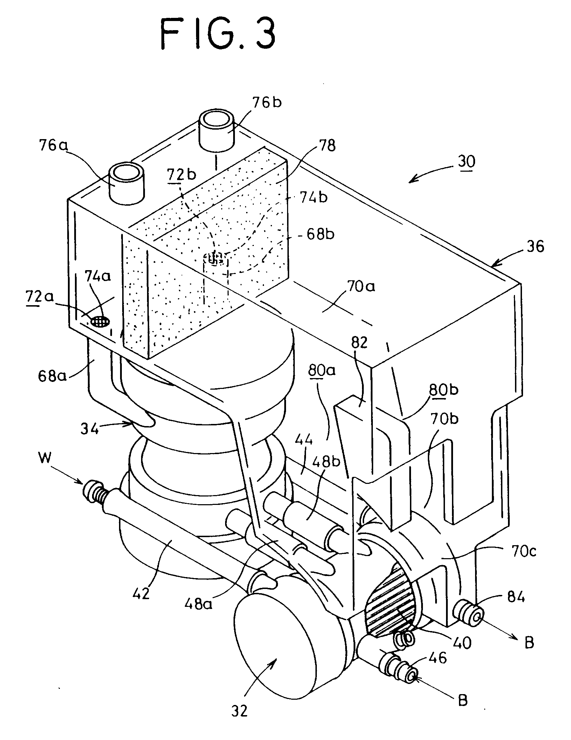

As shown in FIGS. 2 and 3, an extracorporeal blood circulating apparatus according to the present invention has a unitary apparatus assembly 30 comprising a heat exchanger 32, an artificial lung 34, and an open-type blood reservoir 36.

The heat exchanger 32 includes a tubular housing 38 disposed with its longitudinal axis lying horizontally and accommodating therein a number of heat exchanger pipes 40 extending longitudinally in the housing 38. A temperature control fluid such as warm or cold water W is supplied from a water port 42 to the pipes 40, and then discharged from the pipes 40 via a water port 44. A blood supply port 46 is disposed on a lower side peripheral portion of the housing 38 in communication with a heat exchanger chamber 45 defined between the inner peripheral wall surface of the housing 38 and the outer peripheral wall surfaces of the pipes 40. The blood supply port 46 is positioned substantially centrally in the longitudinal direction of the housing 38. Two joint tubes 48a, 48b are coupled to an upper side peripheral portion of the housing 38 in communication with the heat exchanger chamber 45, the joint tubes 48a, 48b being substantially diametrically opposite to the blood supply port 46. The joint tubes 48a, 48b are spaced from each other or disposed on opposite sides in the longitudinal direction of the housing 38. Blood B flowing from the blood supply port 46 into the heat exchanger 32 is heated or cooled to a prescribed temperature while it is passing around the pipes 40. Thereafter, the blood B is supplied via the joint tubes 48a, 48b (see FIG. 3) to the artificial lung 34.

The artificial lung 34 serves to remove carbon dioxide from the blood B and add oxygen to the blood B. The artificial lung 34 includes a multiplicity of hollow filamentary membranes 52 bundled and stored in a substantially cylindrical housing 50 with a constricted central portion. On the upper and lower ends of the housing 50, there are mounted partitions 54a, 54b holding the opposite ends of the hollow filamentary membranes 52. The housing 50 and the partitions 54a, 54b jointly define a space surrounded thereby and housing the hollow filamentary membranes 52 therein, the space having a lower end communicating with the heat exchanger 32 through the joint tubes 48a, 48b. This space serves as a gas exchanging chamber 56 through which the blood B flows. Covers 58a, 58b are also mounted on the upper and lower ends of the housing 50. The partition 54a and the cover 58a define a gas supply chamber 60 therebetween, and the partition 54b and the cover 58b define a gas discharge chamber 62 therebetween. The hollow filamentary membranes 52 are supplied with a gas A containing oxygen from a gas inlet port 64 via the gas supply chamber 60. The gas A delivered from the hollow filamentary membranes 52 is discharged through the gas discharge chamber 62 from a gas outlet port 66.

The gas exchanging chamber 56 in the artificial lung 34 has an upper end communicating with the blood reservoir 36 through two L-shaped joint tubes 68a, 68b each having a horizontal portion extending from the gas exchanging chamber 56 and a vertical portion extending upwardly from the end of the horizontal portion over a certain length. The blood reservoir 36 has a bottom plate composed of first through third steps 70a, 70b, 70c. The joint tubes 68a, 68b have blood inlet ports 72a, 72b, respectively, opening at the first uppermost step 70a on its lateral sides through blood dispersing mesh screens 74a, 74b, respectively, for smoothing the blood flow flowing therethrough. The blood reservoir 36 is made of a transparent plastic material. The blood reservoir 36 has a pair of fluid inlet ports 76a, 76b above the blood inlet ports 72a, 72b, respectively, for adding various fluids, in particular drugs such as a vasodilatator drug, an anticoagulant, and the like to the blood B.

A urethane anti-foaming member 78 is disposed on the first step 70a of the blood reservoir 36 near the blood inlet ports 72a, 72b. A partition 82 is mounted substantially centrally on the second step 70b and divides the blood storage space on the second step 70b into a first blood storage region 80a and a second blood storage region 80b for preventing the blood surface from being subjected to resonant vibration. The third lowermost step 70c is coupled to a blood outlet port 84 for supplying the blood B stored in the blood reservoir 36 into the human body. In operation, the blood outlet port 84 is coupled to the human body through a pump (not shown) which delivers the blood at a prescribed pulse rate. In the case, the pump may be a rotary type or peristaltic finger type.

The extracorporeal blood circulating apparatus of the present invention is basically of the above structure. Now, the operation and advantages of the extracorporeal blood circulating apparatus will be described below.

The blood B coming from the blood supply port 46 into the heat exchanger 32 is controlled at a desired temperature in the heat exchanger chamber 45. More specifically, the pipes 40 are disposed horizontally in the heat exchanger chamber 45 with the water W at a prescribed temperature flowing through the pipes 40. The blood B in the chamber 45, while passing along the outer peripheral surfaces of the pipes 40, is controlled at the desired temperature, and thereafter supplied through the joint tubes 48a, 48b to the artificial lung 34.

The heat exchanger 32 is incorporated in the assembly 30 with its longitudinal axis lying horizontally. The blood B enters the heat exchanger chamber 45 from the blood supply port 46 on the lower side portion of the housing 38 for temperature control, and is thereafter delivered through the joint tubes 48a, 48b into the aritificial lung 34. The difference in height between the blood supply port 46 and the joint tubes 48a, 48b is considerably smaller than the difference in height between the blood inlet port 8 and the joint tubes 12 shown in FIG. 1. Therefore, the blood B flowing in from the blood supply port 46 can be supplied via the joint tubes 48a, 48b into the artificial lung 34 without substantially reducing the potential energy of the blood B. As a result, no pump is required to be coupled upstream of the blood supply port 46, and the blood B can be supplied to the artificial lung 34 and the blood reservoir 36 only under the blood head.

The blood B ingresses into the heat exchanger chamber 45 through the blood supply port 46 communicating with the lower portion of the heat exchanger chamber 45, and is fed to the artificial lung 34 via the joint tubes 48a, 48b communicating with the upper portion of the heat exchanger chamber 45. Therefore, a highly efficient heat exchange is effected on the blood B which has entered the heat exchanger chamber 45.

The blood B fed from the heat exchanger 32 via the joint tubes 48a, 48b into the artificial lung 34 enters the gas exchanging chamber 56 surrounded by the housing 50 in which oxygen is added to the blood B and carbon dioxide is removed from the blood B. More specifically, the gas A containing oxygen is supplied into the bundled hollow filamentary membranes 52 in the housing 50 from the gas inlet port 64 through the gas supply chamber 60. Oxygen and carbon dioxide are exchanged in the blood B through the hollow filamentary membranes 52. The gas A containing carbon dioxide is discharged via the gas discharge chamber 62 from the gas outlet port 66.

The blood B to which oxygen has been added in the artificial lung 34 then flows through the joint tubes 68a, 68b and the blood inlet ports 72a, 72b into the blood reservoir 36. As described above, the heat exchanger 32 is substantially horizontally disposed. Therefore, the blood inlet ports 72a, 72b can be held in a much lower position than the patient. Accordingly, the blood B can easily flow into the blood reservoir 36 only under the head of the blood from the patient. The joint tubes 68a, 68b are of the L shape including a horizontal portion extending from the upper end of the gas exchanging chamber 56 and a vertical portion extending upwardly from the horizontal portion over a certain length. Therefore, the blood B flowing out of the gas exchanging chamber 56 under the pressure developed by the pump (not shown) or the blood head produced by the higher human body position flows upwardly through the L-shaped joint tubes 68a, 68b, during which time the kinetic energy of the blood B is reduced by an increase in the positional energy thereof, thereby resulting in a reduction in the speed of flow of the blood B. As a consequence, the blood B is allowed to flow smoothly into the blood reservoir 36. The blood B is dispersed by the mesh screens 74a, 74b mounted respectively in the blood inlet ports 72a, 72b, and then stored into the blood reservoir 36 through the urethane anti-foaming member 78.

The blood B stored in the blood reservoir 36 is then intermittently or pulsatively delivered out to the human body by the pump (not shown) coupled to the blood outlet port 84. The surface level 86 of the blood B stored in the blood reservoir 36 would be subjected to resonant vibration due to intermittent delivery of the blood B by the pump. However, such resonant vibration is highly effectively suppressed by the partition 82 on the second step 70b in the blood reservoir 36. More specifically, the partition 82 located in the blood reservoir 36 is effective in making the system's natural frequency widely different from the pulse rate of the pump, thus suppressing the undesirable resonant vibration. Therefore, the surface level 86 of the blood B is kept calm without violent turbulent motion. Since the fluctuation of the surface level 86 arising from the pulse rate of the pump is small, the height of the surface level 86, i.e., the amount of the blood B stored in the blood reservoir 36 can easily and accurately be checked by a visual inspection. Moreover, inasmuch as any turbulent motion of the blood B in the blood reservoir 36 is effectively suppressed, vibration of the blood B is held to a minimum, and no unwanted vibration is applied to the apparatus.

With the present invention, as described above, the extracorporeal blood circulating apparatus includes the artificial lung and the blood reservoir which are joined to each other. The blood is introduced into the substantially horizontal heat exchanger for blood temperature control, and thereafter is fed to the artificial lung for a gas exchange, followed by storage in the blood reservoir. Since the heat exchanger is substantially horizontally disposed, the difference in height between the incoming stream of blood and the outgoing stream of blood is very small, and hence the difference in height between the patient and the blood outlet ports for delivering the blood from the artificial lung into the blood reservoir is increased. As a consequence, the blood can be fed to and stored in the blood reservoir only under the blood head without requiring a blood pressurizing means such as a pump which should otherwise be coupled upstream of the heat exchanger.

## Claims

1. An extracorporeal blood circulating apparatus comprising a heat exchanger (32) having a blood inlet (46) and a blood outlet (48a, 48b) and an artificial lung (34) having a blood inlet and a blood outlet, said blood outlet of the heat exchanger being held in communication with said blood inlet of the artificial lung, the arrangement being such that blood is introduced into said heat exchanger from the blood inlet thereof for temperature control, then delivered from the blood outlet of the heat exchanger into said artificial lung via the blood inlet thereof for gas exchange in the artificial lung, and thereafter fed out of the blood outlet of said artificial lung,
characterized in that said blood inlet (46) of said heat exchanger (32) is in a lower portion thereof and said blood outlet (48a, 48b) in an upper portion thereof;
said blood inlet of said artificial lung (34) is in a lower portion thereof and said blood outlet in an upper portion thereof; and
the height of said heat exchanger (32) is smaller than the other dimensions so as to make the height difference between the incoming blood and the outgoing blood small.

2. An extracorporeal blood circulating apparatus according to claim 1, characterized in that said heat exchanger (32) comprises a substantially tubular housing (38) having first and second ends and a plurality of heat exchanger pipes (40) disposed in said housing, said heat exchanger pipes extending longitudinally in said housing.

3. An extracorporeal blood circulating apparatus according to claim 2, characterized in that said blood inlet of the heat exchanger (32) is disposed substantially centrally in the lower portion thereof in the longitudinal direction of the heat exchanger, and said blood outlet is disposed in the upper portion on each of opposite ends in the longitudinal direction of the heat exchanger.

4. An extracorporeal blood circulating apparatus according to claim 2, characterized in that said heat exchanger pipes (40) are supplied with a temperature control fluid from the first end of said heat exchanger (32) and discharge the temperature control fluid from the second end of said heat exchanger.

## Patentansprüche

1. Gerät für außerkörperliche Blutumwälzung, umfassend einen Wärmetauscher (32) mit einem Bluteinlaß (46) und einem Blutauslaß (48a, 48b) sowie eine künstliche Lunge (34) mit einem Bluteinlaß und einem Blutauslaß, wobei der Blutauslaß des Wärmetauschers mit dem Bluteinlaß der künstlichen Lunge in Verbindung gehalten ist, und wobei die Anordnung so getroffen ist, daß Blut vom Bluteinlaß des Wärmetauschers für Temperaturrregelung oder -einstellung in den Wärmetauscher eingeführt, sodann vom Blutauslaß des Wärmetauschers für einen Gasaustausch in der künstlichen Lunge über deren Bluteinlaß in die künstliche Lunge geliefert und anschließend vom Blutauslaß der künstlichen Lunge nach außen gefördert wird,
dadurch gekennzeichnet, daß sich der Bluteinlaß (46) des Wärmetauschers (32) in einem unteren Abschnitt desselben und der Blutauslaß (48a, 48b) in einem oberen Abschnitt desselben befinden,
der Bluteinlaß der künstlichen Lunge (34) in einem unteren Abschnitt derselben und der Blutauslaß in einem oberen Abschnitt derselben liegen und
die Höhe des Wärmetauschers (32) kleiner ist als die anderen Maße oder Abmessungen, so daß der Höhenunterschied zwischen dem einströmenden Blut und dem ausströmenden Blut klein ist.

2. Gerät für außerkörperliche Blutumwälzung nach Anspruch 1, dadurch gekennzeichnet, daß der Wärmetauscher (32) ein im wesentlichen rohrförmiges Gehäuse (38) mit ersten und zweiten Enden sowie eine Vielzahl von im Gehäuse angeordneten Wärmetauscherrohren (40) umfaßt, wobei die Wärmetauscherrohre im Gehäuse in Längsrichtung verlaufen.

3. Gerät für außerkörperliche Blutumwälzung nach Anspruch 2, dadurch gekennzeichnet, daß der Bluteinlaß des Wärmetauschers (32) im wesentlichen zentral in seinem unteren Abschnitt in der Längsrichtung des Wärmetauschers angeordnet ist und der Blutauslaß im oberen Abschnitt an jedem von gegenüberliegenden Enden in der Längsrichtung des Wärmetauschers angeordnet ist.

4. Gerät für außerkörperliche Blutumwälzung nach Anspruch 2, dadurch gekennzeichnet, daß die Wärmetauscherrohre (40) vom ersten Ende des Wärmetauschers (32) her mit einem Temperaturregelfluid beschickt werden und das Temperaturregelfluid am zweiten Ende des Wärmetauschers austragen oder abführen.

## Revendications

1. Appareil de circulation sanguine extracorporelle comprenant un échangeur de chaleur (32) comportant une admission de sang (46) et une sortie de sang (48a, 48b) et un poumon artificiel (34) comportant une admission de sang et une sortie de sang, ladite sortie de sang de l'échangeur de chaleur étant maintenue en communication avec ladite admission de sang du poumon artificiel, la disposition étant telle que du sang est introduit dans ledit échangeur de chaleur par son admission de sang en vue de la régulation de la température, puis est apporté par la sortie de sang de l'échangeur de chaleur audit poumon artificiel par son admission de sang, en vue de l'échange gazeux dans le poumon artificiel, puis est évacué par la sortie de sang dudit poumon artificiel,
caractérisé en ce que ladite admission de sang (46) dudit échangeur de chaleur (32) se trouve dans sa partie inférieure et que ladite sortie de sang (48a, 48b) se trouve dans sa partie supérieure;
que ladite admission de sang dudit poumon artificiel (34) se trouve dans sa partie inférieure et que ladite sortie de sang se trouve dans sa partie supérieure et
que la hauteur dudit échangeur de chaleur (32) est inférieure aux autres dimensions, de façon à minimiser la différence de hauteur entre le sang entrant et le sang sortant.

2. Appareil de circulation sanguine extracorporelle selon la revendication 1, caractérisé en ce que ledit échangeur de chaleur (32) comprend un boîtier sensiblement tubulaire (38) comportant une première et une seconde extrémité et une pluralité de tubes échangeurs de chaleur (40) disposés dans ledit boîtier, lesdits tubes échangeurs de chaleur s'étendant longitudinalement dans ledit boîtier.

3. Appareil de circulation sanguine extracorporelle selon la revendication 2, caractérisé en ce que ladite admission de sang de l'échangeur de chaleur (32) est disposée de façon sensiblement centrale dans sa partie inférieure, dans la direction longitudinale de l'échangeur de chaleur et que ladite sortie de sang est disposée dans la partie supérieure, sur chacune des extrémités opposées, dans la direction longitudinale de l'échangeur de chaleur.

4. Appareil de circulation sanguine extracorporelle selon la revendication 2, caractérisé en ce que lesdits tubes échangeurs de chaleur (40) sont alimentés en un liquide de réglage de la température par la première extrémité dudit échangeur de chaleur (32) et évacuent le liquide de réglage de la température par la seconde extrémité dudit échangeur de chaleur.
